# EUROPEAN PATENT APPLICATION

(11) **EP 3 103 504 A1**
(43) Date of publication of application: **14.12.2016**
(21) Application number: 16163133.8
(22) Date of filing: 31.03.2016
(51) Int. Cl.: A61M 37/00

(54) **A TYPE OF RAPID EXCHANGE OF TATTOOING NEEDLE ASSEMBLY**

(30) Priority: 10.06.2015 CN 201520395864 U
(71) Applicant: Buddha Brand Tattoo Limited, Christchurch, Dorset BH23 1EF (GB)
(72) Inventor: Zhao, Juan, Huatian Village Xinging County Hunan 422714 (CN)
(74) Representative: chapman + co

(57) **Abstract**

A type of assembly that allows the rapid exchange of tattooing needles: a box shaped needle seat 1 that is open at one end, a push-pull lock plate 4 matching the needle seat and located between an upper clamp board 2 and a lower clamp board 3, protective needle sleeves 5 and tattooing needle assemblages 6, there being a number of rows of circular holes located on the aforementioned upper clamp board, there being a number of elongated lock holes, corresponding to but being smaller in diameter than the circular holes, located in the push-pull lock plate, said holes being fitted with protective needle sleeves including a main body 9 and a base 10, said push-pull lock plate functioning in conjunction with the lock holes and limiting grooves 11 to lock the protective needle sleeves securely to the needle seat.

## Description

### Technical Field

This invention relates to a type of needle change over assembly, in particular it is a type of assembly that allows the rapid change over of tattooing needles.

### Background Technology

Commonly encountered tattooing assemblies consist of a tattooing unit, a tattooing unit shank, a needle aperture and the tattooing needle. Before use, the operator must assemble these components. The tattooing unit shank connects with the tattoo unit, the tattooing needle passing through the hollow centre of the shank. The needle aperture is either connected to the shank or is integrated in the shank. The tip section of the tattooing needle relies on the needle aperture to act as a front mount and a guide component, the rear end of the tattoo needle connecting to a component within the tattooing unit that causes the back and forth motion, the component causing the back and forth motion which acts as the mount for the rear end of the tattooing needle also causes the backwards and forwards motion of the tattooing needle. For safety's sake, the tattooing needle is disposable, whilst disposable needle apertures and shanks are also widely used.

### Invention Content

In the light of the drawbacks of current technology, this invention provides a type of assembly with a simple structure, which allows the secure location of tattooing needles and which additionally has functionality that allows the rapid change over of tattooing needle assemblages.

The technical scheme of this invention is: a type of assembly that allows the rapid change over of tattooing needles, whereby: this includes a box shaped needle seat that is open at one end, a push-pull lock plate matching the needle seat and located between an upper clamp board and lower clamp board, protective needle sleeves and tattooing needle assemblages, there being a number of rows of circular holes located on the aforementioned upper clamp board, there being a number of elongated lock holes, corresponding to but smaller in diameter than the circular holes, located in the push-pull lock plate, said circular holes being fitted with protective needle sleeves, the protective needle sleeves including a main body and a base, the bottom section of said main body connecting with the base, in addition to which there are limiting grooves between the main body and the base, said push-pull lock plate functioning in conjunction with the lock holes and limiting grooves to lock the protective needle sleeves securely to the needle seat, the round holes in the lower clamp board possessing fixing apertures that correspond to the bases, the tattooing needle assemblages being in a sleeved arrangement within the aforementioned protective needle sleeves.

When the push-pull lock plate is closed on the needle seat, the space between the lock hole and the round hole surrounding it becomes smaller, the limiting surface of the lock hole and limiting groove of the protective needle sleeve connecting together tightly.

There are needle seat feet located at the four corners of the aforementioned needle seat, these needle seat feet being either magnetic feet or suction feet.

There are three rows of round holes located on the aforementioned upper clamp board, there being between 3 to 4 round holes in each row.

The aforementioned protective needle sleeves possess ribs located evenly around the external circumference of the main body, the outside of those ribs coming into perfect contact with the inside of the round holes.

The cross-sectional shape of the aforementioned base is circular with a flat notch on one side, the surface corresponding to that flat notch fitting against the locating surface of the lower clamp board.

The aforementioned tattooing needle assemblage includes a main shell, a needle component and a needle aperture within that main shell, one end of the main shell connecting with the needle aperture, the other end connecting with an end cap, there being a through-hole in that end cap; on one side within the needle aperture there is a needle guide wall, the needle component extending outside of the main shell through the through-hole, the other end fitting closely with the needle guide wall and extending beyond the needle aperture; there being six clamps arranged evenly around the circumference of the external circular surface of the main shell, there being a pair of clips on the exterior of the hollow tubular shaft connecting the main shell and the tattooing unit, the main shell connecting to the tattooing unit via these clips, being connecting in a sleeved arrangement to the protective needle sleeve by the clamps.

The advantages of this invention are: by an arrangement which includes the location of a number of rows of round holes on a needle seat where protective needle sleeves and tattooing needle assemblages may be placed, relying on a push-pull lock plate onto which protective needle sleeves may be clipped or detached, the securing holes in the lower clamp board restricting the rotation of the protective needle sleeve, this scheme allows completion of the tattooing needle removal action by twisting of the tattooing unit to a certain angle; the interaction between the ribs on the outside of the protective needle sleeve and the round holes is beneficial in that it increases the stability of the protective needle sleeve on the needle seat, preventing excessive wobbling from occurring during the process of the changeover of the tattooing needle.

### Appended Diagrams Description

Figure 1 is a structural representation of the closed push-pull lock plate of the rapid change over tattooing needle assembly to which this invention relates;
Figure 2 is a structural representation of the closed push-pull lock plate of the rapid change over tattooing needle assembly to which this invention relates
Figure 3 is a front view of the arrangement of the needle seat and push-pull lock plate of this invention;
Figure 4 is a top view of the arrangement of the needle seat and push-pull lock plate of this invention;
Figure 5 is a structural representation of the protective needle sleeve to which this invention relates;
Figure 6 is a cross-sectional structural view of the protective needle sleeve to which this invention relates;
Figure 7 is a top view of the protective needle sleeve to which this invention relates;
Figure 8 is a bottom view of the protective needle sleeve to which this invention relates;
Figure 9 is a structural view of the tattooing needle assemblage to which this invention relates;
Where: 1- needle seat, 2 - upper clamp board, 3 - lower clamp board, 4 - push-pull lock plate, 5 - protective needle sleeve, 6 -tattooing needle assemblage, 7 - round hole, 8-lock hole, 9-main body, 10-base, 11-limiting groove, 12 - needle seat foot, 13 - rib, 14 - flat notch, 15 - main shell, 16 - needle component, 17 - needle aperture, 18 - end cap, 19-clamp, 20-clampgroove, 21-clip.

### Detailed Implementation

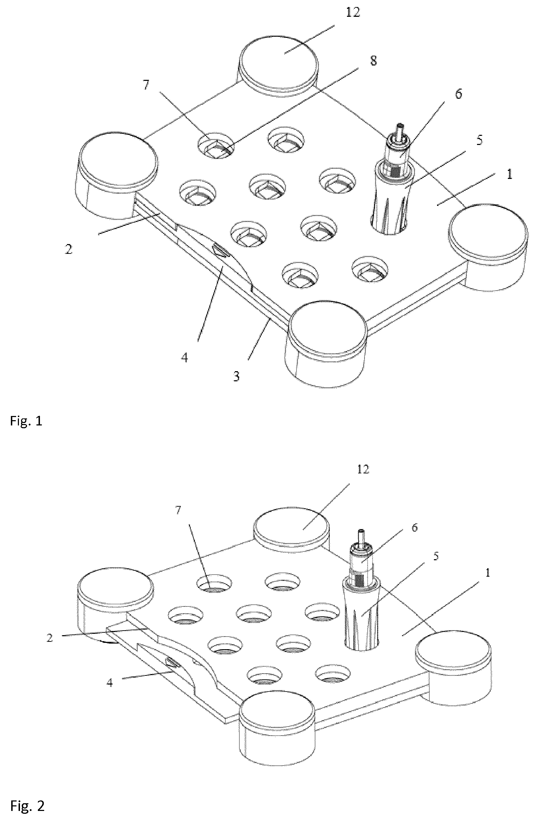

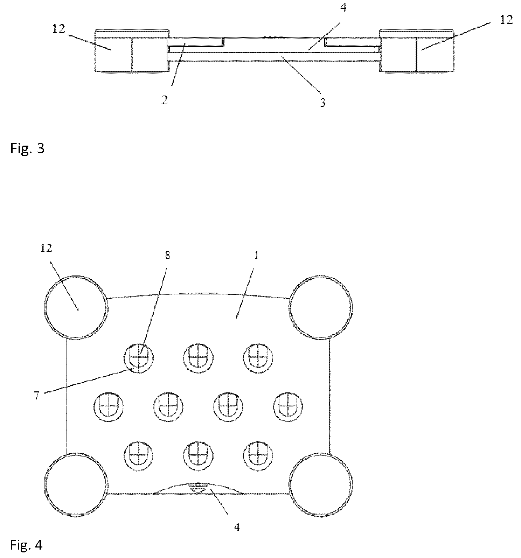

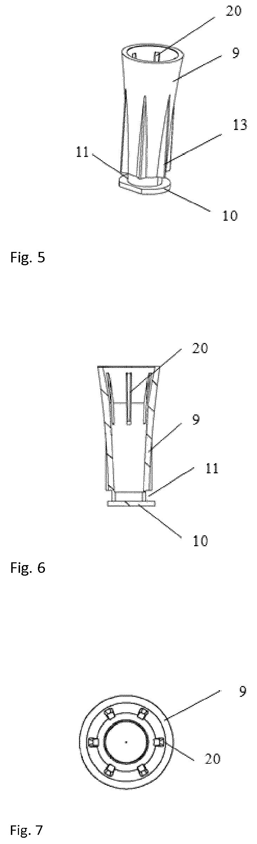

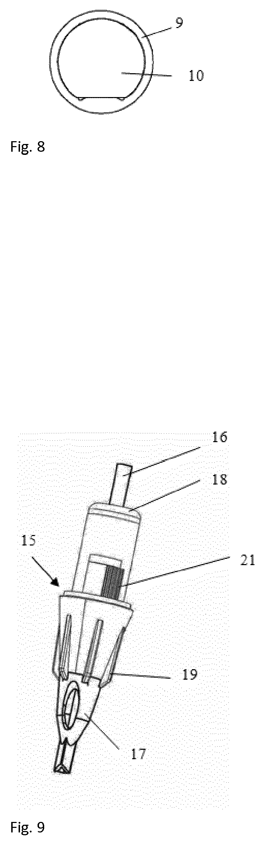

Taken with the appended diagrams the following provides a more detailed description of a detailed implementation of this invention:
As indicated in figures 1-9, a type of assembly that allows the rapid change over of tattooing needles, whereby: this includes a box shaped needle seat 1 that is open at one end, a push-pull lock plate 4 matching the needle seat and located between an upper clamp board and lower clamp board (2, 3), protective needle sleeves 5 and tattooing needle assemblages 6, there being a number of rows of circular holes 7 located on the aforementioned upper clamp board 2, there being a number of elongated lock holes 8, corresponding to but smaller in diameter than the circular holes 7, located in the push-pull lock plate 4, said circular holes 7 being fitted with protective needle sleeves 5, the protective needle sleeves 5 including a main body 9 and a base 10, the bottom section of said main body 9 connecting with the base 10, in addition to which there are limiting grooves 11 between the main body 9 and the base 10, said push-pull lock plate 4 functioning in conjunction with the lock holes 8 and limiting grooves 11 to lock the protective needle sleeves 5 securely to the needle seat 1, the round holes 7 in the lower clamp board 3 possessing fixing apertures that match up with the bases 10, the tattooing needle assemblages 6 being in a sleeved arrangement within the aforementioned protective needle sleeves 5.

When the push-pull lock plate 4 is closed on the needle seat 1, the spaces between the lock holes 8 and the round holes 7 surrounding them become smaller, the limiting surface of the lock hole 8 and limiting groove 11 of the protective needle sleeve 5 connecting together tightly.

There are needle seat feet 12 located at the four corners of the aforementioned needle seat 1, these needle seat feet 12 being either magnetic feet or suction feet, magnets or suction feet allowing the secure fixing of the needle seat 1 to a work surface.

There are three rows of round holes 7 located on the aforementioned upper clamp board 2, there being between 3 to 4 round holes 7 in each row.

The aforementioned protective needle sleeves 5 possess ribs 13 located evenly around the external circumference of the main body 9 , the outside of those ribs 13 coming into perfect contact with the inside of the round holes 7.

The cross-sectional shape of the aforementioned base 10 is circular with a flat notch 14 on one side, the surface corresponding to that flat notch 14 fitting against the locating surface of the lower clamp board 3.

The aforementioned tattooing needle assemblage 6 includes a main shell 15, a needle component 16 and a needle aperture 17 within that main shell 15, one end of the main shell 15 connecting with the needle aperture 17, the other end connecting with an end cap 18, there being a through-hole in that end cap 18; on one side within the needle aperture 17 there is a needle guide wall, the needle component 16 extending outside of the main shell 15 through the through-hole, the other end fitting closely with the needle guide wall and extending beyond the needle aperture 17; there being six clamps 19 arranged evenly around the circumference of the external circular surface of the main shell 15, there being a pair of clips 21 on the exterior of the hollow tubular shaft connecting the main shell 15 and the tattooing unit, the main shell 15 connecting to the tattooing unit via these clips 21, connecting in a sleeved arrangement to the protective needle sleeve 5 by the clamps 19, the clamp grooves 20 on the inside of the protective needle sleeve 5 matching the clamps 19 of the main shell 15.
By an arrangement which includes the location of a number of rows of round holes 7 on the needle seat 1 where protective needle sleeves 5 and tattooing needle assemblages 6 may be placed, relying on a push-pull lock plate 4 onto which protective needle sleeves 5 may be clipped or detached, the securing holes in the lower clamp board 3 restricting the rotation of the protective needle sleeve 5, this scheme allows completion of the tattooing needle removal action by twisting of the tattooing unit to a certain angle; the interaction between the ribs 13 on the outside of the protective needle sleeve 5 and the round holes 7 being beneficial in that it increases the stability of the protective needle sleeves 5 on the needle seat 1, preventing excessive wobbling from occurring during the process of the changeover of the tattooing needle.

This scheme is forward looking, is hygienic to use and innovative. For instance: before a tattooist starts work on a piece, they can select 10 different types of tattooing needle assemblage for use and place these in the needle seat, the type of feet used for the needle seat, such as sucker feet or magnets, allowing it to be securely fixed on a glass surface or steel surface. The tattooist only needs to reach out with one hand and use a light clockwise twisting motion through a 20 degree angle on the exposed end of the needle type that he prefers, to be able to combine the needle and tattooing unit and detach them from the protective needle sleeve in a contactless manner in less than 5 seconds. Another aspect of this is that, when the tattooist wishes to change the type of needle, by placing the tattooing needle assemblage back in the original protective needle sleeve from which it was removed, then by twisting it lightly anticlockwise through a 20 degree angle, they can leave the needle within the protective needle sleeve in a contactless manner thus separating it from the tattooing unit.

The above mentioned implementation and description are simply to provide a description of the principles behind this invention in addition to an optimal implementation, and where they do not depart from the spirit and scope of this invention, it would be possible to make many modifications or improvements to this invention, and these modifications and improvements shall all fall within the scope of protection claimed for this invention.

## Claims

1. A type of rapid change over tattooing needle assembly, whereby this includes a box shaped needle seat that is open at one end, a push-pull lock plate matching the needle seat and located between an upper clamp board and a lower clamp board, protective needle sleeves and tattooing needle assemblages, there being a number of rows of circular holes located on the aforementioned upper clamp board, there being a number of elongated lock holes, corresponding to but smaller in diameter than the circular holes, located in the push-pull lock plate, said circular holes being fitted with protective needle sleeves, the protective needle sleeves including a main body and a base, the bottom section of said main body connecting with the base, in addition to which there are limiting grooves between the main body and the base, said push-pull lock plate functioning in conjunction with the lock holes and limiting grooves to lock the protective needle sleeves securely to the needle seat, the round holes in the lower clamp board possessing fixing apertures that correspond to the bases, the tattooing needle assemblages being in a sleeved arrangement within the aforementioned protective needle sleeve.

2. The type of rapid change over tattooing needle assembly according to claim 1, whereby when the push-pull lock plate is closed on the needle seat, the space between the lock hole and the round hole surrounding it becomes smaller, the limiting surface of the lock hole and limiting groove of the protective needle sleeve connecting together tightly.

3. The type of rapid change over tattooing needle assembly according to claim 1, whereby there are needle seat feet located at the four corners of the aforementioned need seat, these needle seat feet being either magnetic feet or suction feet.

4. The type of rapid change over tattooing needle assembly according to claim 1, whereby there are three rows of round holes located on the aforementioned upper clamp board, there being between 3 to 4 round holes in each row.

5. The type of rapid change over tattooing needle assembly according to claim 1, whereby the aforementioned protective needle sleeves possess ribs located evenly around the external circumference of the main body, the outside of those ribs coming into perfect contact with the inside of the round holes.

6. The type of rapid change over tattooing needle assembly according to claim 1, whereby the cross-sectional shape of the aforementioned base is circular with a flat notch on one side, the surface corresponding to that flat notch fitting against the locating surface of the

7. The type of rapid change over tattooing needle assembly according to claim 1, whereby the aforementioned tattooing needle assemblage includes a main shell, a needle component and a needle aperture within that main shell, one end of the main shell connecting with the needle aperture, the other end connecting with an end cap, there being a through-hole in that end cap; on one side within the needle aperture there is a needle guide wall, the needle component extending outside of the main shell through the through-hole, the other end fitting closely with the needle guide wall and extending beyond the needle aperture; there being six clamps arranged evenly around the circumference of the external circular surface of the main shell, there being a pair of clips on the exterior of the hollow tubular shaft connecting the main shell and the tattooing unit, the main shell connecting to the tattooing unit via these clips, being connecting in a sleeved arrangement to the protective needle sleeve by the clamps.
